# EUROPEAN PATENT APPLICATION

(11) **EP 4 091 605 A1**
(43) Date of publication of application: **23.11.2022**
(21) Application number: 21174652.4
(22) Date of filing: 19.05.2021
(51) Int. Cl.: A61K 9/28, A61K 31/00

(54) **PHARMACEUTICAL TABLET COMPRISING ACETYLSALICYLIC ACID**

(71) Applicant: G.L. Pharma GmbH, 8502 Lannach (AT)
(72) Inventor: LEITNER, Joachim, 8502 Lannach (AT)
(74) Representative: SONN Patentanwälte OG

(57) **Abstract**

The present invention relates to a pharmaceutical tablet suitable for oral administration, comprising (i) a tablet core comprising acetylsalicylic acid (ASA) or a pharmaceutically acceptable salt thereof and excipients, and (ii) an enteric coating over said core; wherein the content of the excipients is in the range of 20 wt.-% to 30 wt.-% with respect to the total weight of the pharmaceutical tablet. The present invention further relates to a method for manufacturing said pharmaceutical tablet.

## Description

The present invention relates to a pharmaceutical tablet suitable for oral administration comprising acetylsalicylic acid or a pharmaceutically acceptable salt thereof, and a method for manufacturing the same.

Acetylsalicylic acid (ASA) is a widely recognized and effective analgesic. This drug, including its salts and derivatives (e.g. its aluminum salt or anhydride), is used as nonsteroidal anti-inflammatory drug (NSAID) for both acute and long-term pain or inflammation. It further has an antipyretic (fever-reducing) effect and possesses antiplatelet activity that helps reducing the risk of blood clots. Therefore, it is also employed in the treatment of acute cardiovascular events like myocardial infarction, transient ischemic attack (TIA) or stroke and in the secondary prevention of these events to reduce the risk of recurrent events. Further, it is thought to reduce the risk of certain types of cancer or first cardiovascular events in patients with a high risk (Brigden M., Smith R. E., Can Med Assoc J, 1997, 156, 1025-8; Aronoff D. M., Neilson E. G., Am J Med, 2001, 111 (4), 304-15; Undas et al., J Thromb Haemost, 2014, 12, 1776-87).

ASA is very hygroscopic, leading to quick degradation in a humid environment. It is a weak acid and accumulates in the stomach lumen due to an ion-trapping mechanism of stomach mucosa cells, causing gastric irritation. Most orally administered ASA drugs are therefore enteric-coated, which is commonly achieved by formulating enteric-coated tablets, enteric-coated granules within capsules, or by means of a multiple-unit pellet system (MUPS).

ASA has been marketed in many different delivery forms, such as compressed tablets (e.g. Thrombo ASS^{®} f/c tablets, Aspirin^{®} tablets), powders (e.g. Goody's^{®} and BC^{®} powders) and effervescent tablets (e.g. Alka-Seltzer^{®} tablets).

Tablet compositions offer many advantages, such as facile handling of the product, chemical and physical stability, portability (and thus allowing ready availability to the patients when needed), aesthetic acceptability and precision of dosage (i.e. consistent and accurate dosages of the active pharmaceutical ingredient are ensured).

A dry coated tablet having an inner core and an outer layer, with the inner core being an enteric-coated tablet containing ASA, is for instance disclosed in EP 2 785 331 B1.

In EP 3 027 176 B1, pharmaceutical tablets comprising a core and an external layer are disclosed, with the core comprising ASA or a pharmaceutically acceptable salt thereof, the external layer comprising clopidogrel or a pharmaceutically acceptable salt thereof, and the core being separated from the external layer by a three-layer coating.

EP 0 011490 B1 relates to direct compression of a powder mixture to obtain analgesic tablets, hereby obtaining a uniform dispersion of the analgesic medicament throughout the tablet.

In EP 2 811 985 B1, a dosage form comprising a tablet core, preferably in compressed form, that has a coating over its exterior surface and one or more patterns debossed in the tablet surface is disclosed.

Tablet compression and coating are two of the most critical aspects of the manufacturing of a pharmaceutical dosage, as these aspects become immediately apparent to the patient. Tablet compression tools are typically designed to manufacture tablets free of visual defects to the extent that is permitted by the equipment used in the manufacturing process and the composition of the tablet. The production process and the quality and characteristics of the resulting tablet are strongly influenced by the particle size and shape of the used ASA grade.

With regard to the size of ASA particles, fine particles are advantageous in terms of homogeneity of the tablet core, but they decrease the flowability and cause sticking during production, which impedes tabletability, i.e. the formation of a tablet core (or uncoated tablet) via compressing. Contrary, ASA grades with a larger particle size show a good flowability and tabletability but are likely to result in an inhomogeneous, rough surface of the tablet core, which affects mouth feel and swallowability. The quality and/or function of a subsequently applied coating might be negatively affected or even ruined. Thus, only ASA grades with a particle size in a certain range are generally suitable for the manufacture of pharmaceutical tablets to ensure good flowability, good tabletability and a smooth tablet surface.

Apart from the size of ASA particles, their shape imparts a further restriction to suitable ASA grades. ASA tends to crystallize in a needle- or rod-like morphology. Such acicular crystals with a high aspect ratio are undesirable for pharmaceutical manufacturing applications, as they tend to protrude from the surface and may break during tableting, which may cause surface inhomogeneities. Contrary, pharmaceutical particles with a low aspect ratio exhibit very good tabletability, cohesivity, flowability and enable to manufacture a tablet core with a smooth and homogeneous surface. Thus, methods to tailor the shape of ASA crystals via in situ manipulation of crystal habit during growth or via pre-treatment steps, such as roller compacting of acicular ASA crystals, are often applied to form platelet- or flake-shaped crystals. However, these additional processing steps are also accompanied by a significant increase in price.

In consequence, on the one hand, ASA grades for use in the manufacture of pharmaceutical tablets need to be carefully selected with respect to particle size, particle shape and patient comfort, which results in a limited portfolio of suitable ASA grades. On the other hand, in order to overcome said limitations regarding the ASA grades, it is not beneficial to overly increase the amount of excipients for economic reasons as well as regarding tablet size and patient comfort and compliance.

It is thus an object of the present invention to provide a pharmaceutical tablet suitable for oral administration which comprises (i) a tablet core comprising ASA or a pharmaceutically acceptable salt thereof and excipients, and (ii) an enteric coating over said tablet core; and which tablet has an excellent processability, swallowability, smooth surface not a significantly increased tablet size (allowing for good patient comfort and compliance), whilst minimizing the influence of size and shape of ASA particles and thus enabling to choose from a larger portfolio of economically more attractive ASA grades, and whilst keeping the amount of excipients as low as possible to be economically attractive. It is a further object of the present invention to provide a method for the manufacture of such a pharmaceutical tablet.

Starting from this object, it was surprisingly found that an increase (in comparison to commercially available tablets, particularly Aspirin^{®} Protect) of the weight ratio of the excipients that are used together with ASA in the core of the pharmaceutical tablet according to the present invention, in particular up to 20 wt.-% to 30 wt.-% with respect to the total weight of the pharmaceutical tablet,
(i) allows to apply a higher compression force during tableting, which significantly improves tabletability and lowers friability,
(ii) allows to use not only single-tip tooling but also multi-tip tooling, which improves flexibility in terms of tooling selection and increases the output of tablets per hour,
(iii) enables the manufacture of tablets with high hardness, a smooth, homogeneous surface and without significantly increased tablet size, thus ensuring a good mouth feel and swallowability,
(iv) improves the dissolution rate and the release of ASA, and
(v) provides the possibility to choose from a larger portfolio of ASA grades with various particle sizes and shapes, which enhances the flexibility and brings commercial benefits.

The present invention is thus directed to a pharmaceutical tablet suitable for oral administration, comprising
(i) a tablet core comprising acetylsalicylic acid (ASA) or a pharmaceutically acceptable salt thereof and excipients, and
(ii) an enteric coating over said core;
wherein the content of the excipients is in the range of 20 wt.-% to 30 wt.-% with respect to the total weight of the pharmaceutical tablet.

The present invention further relates to a method for manufacturing a pharmaceutical tablet according to the present invention with the content of the excipients in the range of 20 wt.-% to 30 wt.-% with respect to the total weight of the pharmaceutical tablet, comprising the steps of
(i) preparing a mixture comprising acetylsalicylic acid (ASA) or a pharmaceutically acceptable salt thereof and excipients,
(ii) compressing said mixture to obtain a tablet core, and
(iii) applying an enteric coating onto the tablet core to obtain the pharmaceutical tablet according to the present invention.

All of the preferred embodiments of this invention as disclosed in the present application are interrelated, and each preferred embodiment and/or disclosed characteristic feature may be combined with each other and also as any combination of two or more preferred embodiments/characteristic features.

In the pharmaceutical tablet according to the present invention, the active pharmaceutical ingredient (API) is acetylsalicylic acid (ASA) or a pharmaceutically acceptable salt thereof. Within the context of the present invention, the term "ASA" comprises the acid form and all pharmaceutically acceptable salts of ASA likewise. As ASA is usually given orally as the free acid and as many ASA grades comprising the acid form are available, this free acid form is preferably used in the inventive pharmaceutical tablet. The terms "particle(s)" and "crystal(s)" of ASA are interchangeably used within the present application.

In the context of the present application, the term "tablet" relates to pharmaceutical tablets comprising an inner core and a coating, unless it is explicitly stated that uncoated pharmaceutical tablets are referred to. An uncoated pharmaceutical tablet is devoid of any coating.

The term "coating" is intended to refer to the application of a layer onto the whole surface of the core of the pharmaceutical tablet, so that the tablet core is encased by said layer. The term "enteric coating" is intended to refer to a gastro-resistant coating which dissolves only at a pH value of at least 3.5 or above and protect the gastric environment from the ingredient or the composition that it coats.

The term "tabletability" is intended to refer to the processability of a homogeneous powder mixture comprising an active pharmaceutical ingredient (API) and excipients by means of a tableting machine (also referred to as press machine or compression machine). This tableting machine is equipped with a tool having a single tip or multiple tips which compacts the homogeneous powder mixture to form an uncoated tablet, which is generally referred to as tablet core, here of the pharmaceutical tablet according to the present invention.

The term "hardness" describes the resistance to crushing of tablets as measured by the force needed to disrupt said tablets by crushing (Ph. Eur. 2.9.8). The term "hardness" is used synonymously with the terms "crushing force" and "resistance to crushing" within this application.

The term "friability" describes the brittleness of the tablet core, as core dust may lead to leakage of the enteric coating and consequently may cause problems with gastro-resistance (Ph. Eur. 2.9.7). Thus, tablets need to have a low friability to avoid leakage of the enteric coating.

The pharmaceutical tablet according to the present invention comprises a tablet core that comprises ASA and excipients, and an enteric coating over said tablet core that begins to dissolve when the pH value of the surrounding medium is at least 3.5 or above, allowing the disintegration of the tablet core and the release of ASA.

The core of the inventive pharmaceutical tablet comprises at least one, but usually several excipients that serve as flow enhancer, filler and/or binder. The content of the excipients is in the range of 23 wt.-% to 33 wt.-%, preferably in the range of 27.6 wt.-% to 29.6 wt.-%, and particularly preferably amounts to 28.6 wt.-% with respect to the total weight of the tablet core. With respect to the total weight of the inventive pharmaceutical tablet, the content of excipients ranges from 20 wt.-% to 30 wt.-%, preferably from 24.5 wt.-% to 26.5 wt.-%, and particularly preferably amounts to 25.5 wt.-%.

It was surprisingly found that already a small increase of the weight ratio of the excipients of only 20 wt.-% to 30 wt.-% with respect to the total weight of the pharmaceutical tablet and compared to commercially available tablets, particularly Aspirin^{®} Protect, leads to significant benefits. In particular, the use of excipients in a weight ratio according to the present invention surprisingly enables to choose from a larger portfolio of ASA grades with various particle sizes and shapes without compromising the manufacture of the tablet core. This weight ratio of the excipients rather allows to apply a higher compression force during tableting, which significantly improves tabletability and lowers friability. It also enables to use not only a single-tip tooling but also a multi-tip tooling, which improves flexibility in terms of tooling selection, reduces the number of batches and increases the yield. The resulting pharmaceutical tablet according to the present invention comprises a content of excipients within this weight ratio and surprisingly exhibits improved hardness, a smooth, homogeneous surface and an improved dissolution rate, thus a faster release of ASA.

In a preferred embodiment, the core of the pharmaceutical tablet comprises a flow enhancer to improve the flowability and tabletability. Silica, such as anhydrous colloidal silica, precipitated silica or fumed silica, can be employed as flow enhancer, because silica does not only improve the flowability and tabletability but also enhances the hardness and abrasion resistance. Fumed silica is preferably used as flow enhancer due to its ready availability and high purity, making it especially suitable for pharmaceutical use. In a preferred embodiment, Aerosil^{®} 200 with a specific BET surface area of 175 m²/g to 225 m²/g is used.

According to another preferred embodiment of the present invention, the flow enhancer is present in an amount of at least 0.1 wt.-%, preferably in an amount ranging from 0.3 wt.-% to 0.7 wt.-%, particularly preferably in an amount of 0.5 wt.-% with respect to the total weight of the tablet core. With respect to the total weight of the inventive pharmaceutical tablet, the flow enhancer is present in an amount of at least 0.1 wt.-%, preferably in an amount ranging from 0.2 wt.-% to 0.6 wt.-%, particularly preferably in an amount of 0.4 wt.-%. It was found that the use of a flow enhancer in the given amount significantly improves the flowability and tabletability and provides the inventive pharmaceutical tablet with enhanced hardness and abrasion resistance.

In a further preferred embodiment cellulose, such as microcrystalline or powdered cellulose, is employed as filler in the core of the pharmaceutical tablet according to the present invention. Powdered cellulose is particularly preferred because it provides a good tabletability without any sticking. Starch, preferably maize starch, can serve as binder and promotes disintegration and dissolution of the tablet core once the enteric coating has been dissolved. A combination of powdered cellulose and maize starch is preferably used as it provides excellent tabletability, with enhanced elasticity provided by the powdered cellulose and good binding of the powder mixture provided by the maize starch.

Preferably, the amount of powdered cellulose ranges from 9.0 wt.-% to 19.0 wt.-%, preferably from 12.8 wt.-% to 14.8 wt.-% with respect to the total weight of the tablet core. With respect to the total weight of the pharmaceutical tablet according to the present invention, the amount of powdered cellulose may range from 8.0 wt.-% to 18.0 wt.-%, preferably from 11.3 wt.-% to 13.3 wt.-%.

According to another preferred embodiment of the present invention, the amount of maize starch ranges from 9.0 wt.-% to 19.0 wt.-%, preferably from 13.3 wt.-% to 15.3 wt.-% with respect to the total weight of the tablet core. With respect to the total weight of the pharmaceutical tablet according to the present invention, the amount of maize starch may range from 8.0 wt.-% to 18.0 wt.-%, preferably from 11.7 wt.-% to 13.7 wt.-%.

The enteric coating of the inventive pharmaceutical tablet preferably comprises a gastro-resistant polymer, as polymeric barriers are well suitable to prevent the dissolution or disintegration of orally administered drugs in the gastric environment. Preferably, the enteric coating comprises an acrylic polymer, as this polymer class allows an excellent control of the release of ASA in dependence on the pH value, thus guaranteeing the effectiveness of drug treatment. In a preferred embodiment, the gastro-resistant polymer is a copolymer of methacrylic acid and ethyl acrylate, with the two monomeric repeating units preferably being present in the copolymer in a ratio of 1:1. This copolymer does not only provide a good resistance to the gastric environment but also enhances the hardness and toughness of the tablet, with toughness signifying the ability of a material to absorb energy and plastically deform without fracturing.

The gastro-resistant polymer is preferably present in a 30% aqueous dispersion, with the water being aqua purificata which is removed during manufacture of the pharmaceutical tablet. The content of the gastro-resistant polymer in the enteric coating is preferably in the range of 40 wt.-% to 55 wt.-%, more preferably in the range of 45 wt.-% to 50 wt.-%, particularly preferably in the range of 47 wt.-% to 48 wt.-% with respect to the total weight of the enteric coating.

The enteric coating may further comprise an anti-tacking agent such as talc in an amount of 40 wt.-% to 55 wt.-%, preferably in an amount of 45 wt.-% to 50 wt.-%, particularly preferably in an amount of 47 wt.-% to 48 wt.-% with respect to the total weight of the enteric coating, which serves to prevent sticking of the pharmaceutical tablet.

The enteric coating may further comprise a plasticizer such as triethyl citrate in an amount of 4.0 wt.-% to 5.5 wt.-%, preferably in an amount of 4.6 wt.-% to 4.8 wt.-% with respect to the total weight of the enteric coating, which serves as plasticizer to increase the toughness of the enteric coating.

According to a preferred embodiment, the weight of the dry enteric coating ranges from 9 wt.-% to 12 wt.-% and preferably amounts to 10.8 wt.-% with respect to the total weight of the inventive pharmaceutical tablet, which ensures a good resistance to the gastric environment, allows for a smooth and homogeneous surface of the tablet and increases its hardness and toughness.

For manufacturing the pharmaceutical tablet according to the present invention, ASA and the excipients are blended in the weight ratio according to the present invention to form a homogeneous mixture. A sieving step can additionally be applied prior to blending in order to further enhance the homogeneity and flowability of the resulting mixture.

The core of the pharmaceutical tablet according to the present invention has a composition which enables its direct compression. The compression step is carried out with a tableting machine which compacts the homogeneous mixture into a tablet core. It was surprisingly found that the increased weight of excipients improves the tabletability. A higher compression force can be applied which reduces sticking, so that the inventive method is less sensitive to the particle size and shape of the used ASA grade. This opens up the possibility to choose from a larger portfolio of ASA grades, including commercially more attractive ASA grades with an acicular crystal structure.

Further, the increased weight ratio of the excipients according to the present invention, i.e. the increase of the excipient content of only 20 wt.-% to 30 wt.-% with respect to the total weight of the pharmaceutical tablet and compared to commercially available tablets, particularly Aspirin^{®} Protect, and thus the higher applicable compression force allows to manufacture tablets with a smooth, homogeneous surface and a low friability both with a single-tip tool and multi-tip tool, which allows a higher flexibility in terms of tool selection. Using a multi-tip tool further enables to reduce the number of batches and to increase the yield.

In spite of the increased weight of the inventive pharmaceutical tablet, a significant increase of the tablet size is avoided. Such an increase of the tablet size would result in a reduction in the patient's comfort when ingesting the tablet and could affect patient comfort. Instead, the increased weight of the tablet core allows to apply a higher compression force without damaging the equipment. This increases the density of the tablet whilst the tablet size is not significantly increased. Preferably, the increase of the height of the tablet is below 20%, preferably below 16%, particularly preferably below 13%, when increasing the weight ratio of excipients in the tablet core. The increase of the volume of the tablet is below 25%, preferably below 20%, when increasing the weight ratio of excipients in the tablet core. Thus, a good mouth feel and swallowability are maintained. Further, the method for manufacturing a pharmaceutical tablet according to the present invention allows to use the same manufacturing equipment and the same methods of handling and packaging as applied for commercially available tablets. This brings benefits in view of the high importance of standardization in the pharmaceutical industry to minimize manufacturing and packaging costs. As the same tool can be used in the tableting step, the diameter of the pharmaceutical tablet remains unchanged upon increase of the weight of the tablet core.

Following the compression step, an enteric coating is applied onto the tablet core. Various methods may be used, such as spray coating, film coating, dip coating, enrobing or electrostatic deposition. The enteric coating is preferably applied by means of film coating. Care has to be taken to avoid the formation of any defects and to homogeneously coat the entire surface of the smooth tablet core to ensure excellent resistance to the gastric environment. The tablet core obtained by means of the inventive manufacturing method exhibits smoothness and low friability and thus allows for a homogeneous coating to produce the pharmaceutical tablet according to the present invention.

An important factor in the manufacture of pharmaceutical tablets is mouth feel. However, many pharmaceutical ingredients have an unpleasant mouth feel due to chalkiness, grittiness and dryness, especially if the tablet exhibits a rough surface. In terms of ASA, particle size and shape usually strongly influence the surface properties of tablets, also after applying an enteric coating. It was surprisingly found that the method according to the present invention allows for a greater variation of ASA grades with various particle sizes and shapes whilst still yielding pharmaceutical tablets with a homogeneous, smooth coating that is free of visual defects and irregularities. Thus, the present invention opens up the possibility to choose from a larger portfolio of ASA grades available on the market without being limited to only a few grades and/or suppliers.

### Experimental

The following examples are supposed to further illustrate the invention as described within this application without any intention to limit the scope of the invention.

### (1) Composition and manufacture of pharmaceutical tablets

Pharmaceutical tablets according to the present invention (with various ASA grades) and a reference tablet (with the ASA grade Novacyl 3020) were produced (Tab. 1). Further, two commercially available tablets, namely Aspirin^{®} N (uncoated tablet) and Aspirin^{®} Protect with compositions as listed in Tab. 1 were analyzed as reference, with all tablets comprising 100 mg ASA.

In a first step, the ingredients of the tablet core, i.e. ASA and the excipients (maize starch, powdered cellulose and fumed silica, if present) according to the compositions listed in Tab. 1, were weighed by means of a weighing hopper. This was followed by sieving with a rotating sieving machine having round holes with a size of 2.1 mm. Finally, the ingredients were blended in a free fall mixer for 20 min with a rotation speed of 6 rpm to form a homogeneous mixture.

The compression step was carried out with a tableting machine (maximum compression force up to 100 kN, maximum permissible punch load up to 54 kN) to compact the homogeneous mixture into a tablet core. The approximate compression force applied in the tableting step is listed in Tab. 2 in dependence on the weight of the ingredients to form the tablet core (120 mg versus 140 mg) and on the number of tips of the tool (single-tip tool versus four-tip tool). The compression force signifies the force that minimally needs to be applied to form a compressed tablet core with a smooth and homogeneous surface and that maximally can be applied without damaging the equipment.

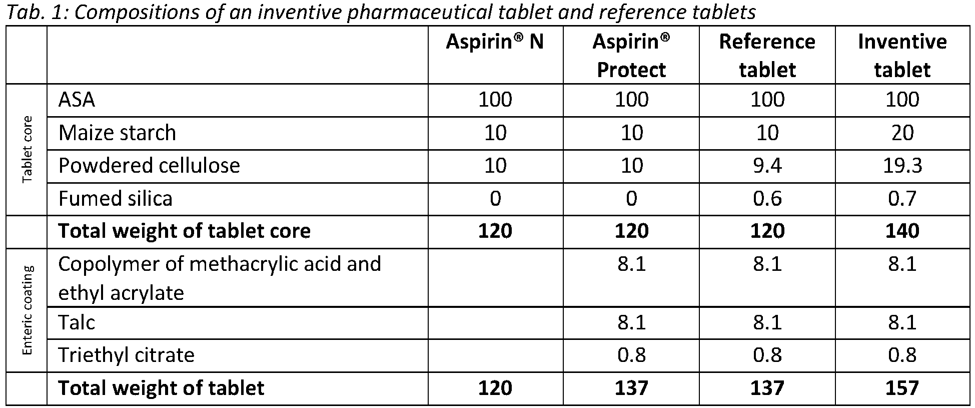

As can be seen from Tab. 2, the achievable hardness values upon increase of the weight of excipients used to form the tablet core from 120 mg to 140 mg (tablet compositions according to Tab. 1). With a mixture having a weight of 120 mg, a tablet core with a smooth surface that resists the mechanical stress during the coating process can only be produced with a single-tip tool, approximately 50 N. When using a four-tip tool, the maximum compression force that can be applied without damaging the equipment is even lower and leads to tablets with 30 N, which is, depending on the used API quality, too low to form a tablet core with a smooth and homogeneous surface.

When increasing the weight of the mixture to form the tablet core to 140 mg, a tablet core with a smooth, even surface and low friability can be manufactured with both a single-tip tool and multi-tip tool, such as with a four-tip tool, as a higher compression force can be applied without damaging the equipment (yielding tablets with approximately 55 N and 45 N for single-tip tool and four-tip tool, respectively). This allows for a higher flexibility in terms of tool selection. The use of a multi-tip tool further enables to reduce the number of batches and to increase the yield.

**Tab. 2: Resulting hardness values in dependence on the weight of the mixture and on the number of tips of the tool used to form the tablet core**

| **Tablet with reference composition** | | **Inventive tablet** | |
|---|---|---|---|
| **single-tip tool** | **four-tip tool** | **single-tip tool** | **four-tip tool** |
| ∼50 N | max. 30 N | ∼ 55 N | ∼ 45 N |

In a following step, an enteric coating comprising the ingredients as listed in Tab. 1 (copolymer of methacrylic acid and ethyl acrylate, talc and triethyl citrate) was applied onto the tablet core by means of film coating to obtain pharmaceutical tablets.

### (2) Influence of ASA grade on manufacture and properties of pharmaceutical tablets

Different ASA grades of various suppliers according to Tab. 3 were employed, of which some grades were pre-treated by means of a pre-processing step to form platelet- or flake-shaped crystals (marked with an asterisk).

**Tab. 3: Grades of acetylsalicylic acid (ASA) of four different suppliers**

| **Shandong Xinhua** | **Novacyl** | **JQC Huayin** | **Andhra Sugars** |
|---|---|---|---|
| 20-60 mesh | 3020 | 20-60 mesh | ASL-40CP |
| 40-80 mesh | 3025 | 40-80 mesh | ASL-25G* |
| 60-120 mesh | 3126* | DC-100* | |
| 30-70 mesh | 3118* | | |

| | | | |
|---|---|---|---|
| *pre-processed ASA grades | | | |

The morphology of several ASA grades is visualized by means of light-microscopic images in Fig. 1a and Fig. 1b (20 times magnified). It is clearly seen that the crystals of ASA grades that were not pre-processed have acicular shapes with a high aspect ratio. This impedes tabletability, as the crystals that protrude from the surface may break during tableting which may consequently lead to surface inhomogeneities. Contrary, the crystals of pre-processed ASA grades (marked with an asterisk) exhibit various shapes. This diverse morphology provides a better tabletability, cohesivity, flowability and facilitates the manufacture of a tablet core with a smooth and homogeneous surface. Pre-processed ASA grades are therefore generally preferred for the manufacture of pharmaceutical tablets in spite of their higher price. However, it was surprisingly found that both pre-processed and non-pre-processed ASA grades can be used to manufacture the pharmaceutical tablet according to the present invention, whilst maintaining excellent processability, tabletability and resulting in a tablet with a smooth and homogeneous surface.

The particle size distribution of several ASA grades as measured via sieve analysis is found in Tab. 4. With regard to the size of ASA particles, fine ASA grades are advantageous in terms of homogeneity of the tablet core but reduce flowability and are likely to cause sticking during production, which impedes tabletability. Contrary, ASA grades with a larger particle size show a good flowability and tabletability but are likely to cause an inhomogeneous, rough surface of the tablet core, which negatively affects mouth feel and hinders swallowability, also after applying a coating.

Several ASA grades were employed in the manufacture of reference tablets with a tablet core having a weight of 120 mg (composition according to Tab. 1). However, the use of non-pre-processed ASA grades caused sticking during tableting already after a few manufacturing cycles. The manufacture of tablet cores comprising the non-pre-processed ASA grade Novacyl 3020 is feasible and suitable for routine production due to the absence of sticking, however, the resulting tablets exhibit a rough surface, which may result in friability and insufficient coating, as well as hindered swallowability. Thus, in general, only pre-processed ASA grades with a particle size in a certain range are suitable for the manufacture of pharmaceutical tablets to ensure good flowability, tabletability, and smoothness of the tablet surface.

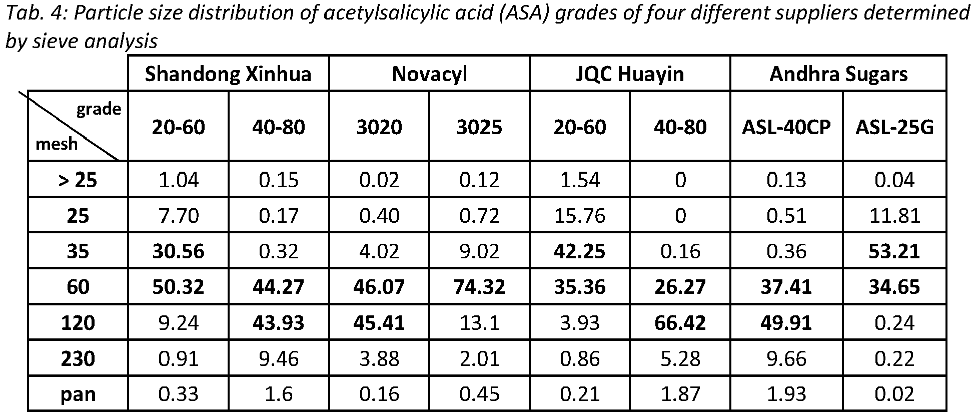

However, it was surprisingly found that increasing the weight ratio of excipients according to the present invention improves the flowability, allows for a good tabletability without any sticking-related issues and results in the manufacture of tablet cores with a smooth and homogeneous surface. The manufacture of tablet cores having an increased weight ratio of excipients is less sensitive to the size and shape of ASA particles, allowing to use a larger portfolio of ASA grades. For example, all of the ASA grades listed in Tab. 3 having various particle size distributions and crystal shapes can successfully be employed to manufacture pharmaceutical tablets according to the present invention.

### (3) Dissolution test of pharmaceutical tablets

A dissolution test of pharmaceutical tablets was performed in a USP apparatus 1 (100 rpm) in accordance with the standards Ph. Eur. 2.9.3, 4.1.3 and 5.17. The pH value of the dissolution medium was set to 1.0 by addition of 750 ml of 0.1 M HCl. The dissolution behavior of the tablets in the acidic medium was observed over a testing period of 120 min. Subsequently, the pH value was raised to 6.8 by addition of a phosphate buffer solution (250 ml of 0.2 M Na₃PO₄). Seven tablets were tested for each ASA grade and taken from the dissolution medium after different durations from 120 min up to 180 min to assess the time-dependent release of ASA.

Fig. 2a and Fig. 2b show the release kinetics of ASA at a pH value of 6.8 for inventive pharmaceutical tablets with various ASA grades, and Aspirin^{®} Protect as reference. The release is expressed as a percentage of the total ASA content, which is 100 mg for all analyzed tablets. In the acidic dissolution medium (pH value of 1.0), the release of ASA was inhibited over the course of 120 min, signifying excellent resistance of all analyzed tablets to the gastric environment. After adjusting the pH value to 6.8, ASA was completely released from all analyzed tablets within 60 min.

As displayed in Fig. 2a, a larger particle size of the used ASA grade generally leads to a faster disintegration (compare for example the release rate of Shandong Xinhua grades: 40-80 mesh > 30-70 mesh > 20-60 mesh). In terms of shape of the ASA crystals, no definite correlation with the release rate is observed. All pharmaceutical tablets according to the present invention that are displayed in Fig. 2a have a tablet core weight of 140 mg whilst the reference Aspirin^{®} Protect has a lower tablet core weight of 120 mg. It can be seen that all inventive pharmaceutical tablets, comprising either pre-processed or non-pre-processed ASA grades with various particle sizes and shapes, perform excellently in the dissolution test and show a faster release of ASA than Aspirin^{®} Protect, clearly showing the improvement of the release rate upon increase of the excipient content.

The release of ASA from an inventive pharmaceutical tablet in comparison to Aspirin^{®} Protect and a reference tablet is displayed in Fig. 2b (compositions of the tablets as listed in Tab. 1). The ASA used to manufacture the inventive pharmaceutical tablet and the reference tablet was Novacyl 3020.

Comparison of the reference tablet with Aspirin^{®} Protect (both having the same weight of tablet core and excipients) shows that the release of ASA from the reference tablet (Novacyl 3020) occurs faster than the release of ASA from Aspirin^{®} Protect.

The increase of the weight ratio of excipients to obtain a pharmaceutical tablet according to the present invention further accelerates the release of ASA, as seen for the tablets comprising Novacyl 3020 with a weight of the tablet core of 120 mg and 140 mg, respectively (Fig. 2b). Evidently, the higher weight ratio of excipients in a pharmaceutical tablet according to the present invention accelerates the break-up of the tablet, hereby enabling a swift disintegration, enhancing the accessible surface area and promoting a more rapid release of the ASA. The inventive pharmaceutical tablet is therefore well suitable to meet the increasing demand of the pharmaceutical industry regarding an improvement of dissolution rates of drugs.

### (4) Geometric dimension of pharmaceutical tablets

Geometric dimensions of an inventive pharmaceutical tablet are shown in Tab. 4 along with dimensions of Aspirin^{®} N (uncoated) and Aspirin^{®} Protect, all comprising 100 mg ASA (compositions as listed in Tab 1). The diameter of Aspirin^{®} N amounts to 7.0 mm, whilst it increases to 7.3 mm after applying an enteric coating (inventive tablet and Aspirin^{®} Protect).

Comparison of geometric dimensions of an inventive pharmaceutical tablet and Aspirin^{®} Protect shows that the increase of the weight of the tablet core from 120 mg to 140 mg by doubling the content of excipients leads to an increase of the tablet height of 12.9% (from 3.1 mm to 3.5 mm), an increase of the surface area of 7.4% (from 135 mm² to 145 mm²) and an increase of the volume of 18.2% (from 110 µl to 130 µl).

**Tab. 4: Geometric dimensions of an inventive pharmaceutical tablet and reference tablets (compositions as listed in Tab. 1)**

| | **Aspirin^{®} N** | **Aspirin^{®} Protect** | **Inventive tablet** |
|---|---|---|---|
| Diameter | 7.0 mm | 7.3 mm | 7.3 mm |
| Height | 2.9 mm | 3.1 mm | 3.5 mm |
| Surface area | 130 mm² | 135 mm² | 145 mm² |
| Volume | 100 µl | 110 µl | 130 µl |

As the geometric dimensions of a pharmaceutical tablet according to the present invention are comparable to the dimensions of commercially available tablets, such as Aspirin^{®} Protect, the same methods of handling and packaging can be used for manufacturing. Further, the same tableting machine with the same tooling can be used, as evidenced by the same diameter of the inventive pharmaceutical tablet and Aspirin^{®} Protect. This is well in line with the high importance of standardization in the pharmaceutical industry. Also, the similar geometric dimensions do not impede swallowability of the tablet and ensure an easy ingestion by the patient.

### (5) Resistance to crushing of pharmaceutical tablets

The resistance to crushing relates to the force required to disrupt tablets by crushing according to Ph. Eur. 2.9.8. The resistance to crushing of an inventive pharmaceutical tablet and references, namely Aspirin^{®} N (uncoated) and Aspirin^{®} Protect, is shown in Tab. 5 (compositions as listed in Tab 1). As can be seen, the required force to disrupt a tablet increases significantly when applying a coating. While Aspirin^{®} N shows a resistance to crushing of only 35-40 N, Aspirin^{®} Protect has a higher resistance to crushing of 65-70 N, which corresponds to an increase of the hardness. It was surprisingly found that due to the increased weight ratio of excipients and the presence of silica, the inventive tablet has an even higher resistance to crushing of 95-100 N and thus a higher hardness than commercially available pharmaceutical tablets, which improves the tabletability. Further, the likelihood for defects formed upon manufacturing of the tablet core is reduced. A smooth, defect-free surface of the tablet core hinders the enteric coating to penetrate into the tablet core, which would consequently impede disintegration and release of ASA.

**Tab. 5: Resistance to crushing (according to Ph. Eur. 2.9.8) of an inventive pharmaceutical tablet and references (compositions as listed in Tab. 1)**

| | **Aspirin^{®} N** | **Aspirin^{®} Protect** | **Inventive tablet** |
|---|---|---|---|
| Resistance to crushing | 35-40 N | 65-70 N | 95-100 N |

## Claims

1. A pharmaceutical tablet suitable for oral administration, comprising
(i) a tablet core comprising acetylsalicylic acid (ASA) or a pharmaceutically acceptable salt thereof and excipients, and
(ii) an enteric coating over said core;
wherein the content of the excipients is in the range of 20 wt.-% to 30 wt.-% with respect to the total weight of the pharmaceutical tablet.

2. The pharmaceutical tablet according to claim 1, wherein the content of the excipients is in the range of 24.5 wt. % to 26.5 wt.-% with respect to the total weight of the pharmaceutical tablet.

3. The pharmaceutical tablet according to claim 1 or 2, wherein the excipients comprise a flow enhancer.

4. The pharmaceutical tablet according to claim 3, wherein the flow enhancer is silica, preferably fumed silica.

5. The pharmaceutical tablet according to claim 3 or 4, wherein the content of the flow enhancer is at least 0.1 wt. % with respect to the total weight of the pharmaceutical tablet.

6. The pharmaceutical tablet according to any of the previous claims, further comprising powdered cellulose and maize starch.

7. The pharmaceutical tablet according to any of the previous claims, wherein the enteric coating comprises an acrylic polymer, preferably a copolymer of methacrylic acid and ethyl acrylate.

8. Method for manufacturing a pharmaceutical tablet according to any of the previous claims, comprising the steps of
(i) preparing a mixture comprising acetylsalicylic acid (ASA) or a pharmaceutically acceptable salt thereof and excipients,
(ii) compressing said mixture to obtain a tablet core, and
(iii) applying an enteric coating onto the tablet core to obtain the pharmaceutical tablet.
